# EUROPÄISCHE PATENTSCHRIFT

(11) **EP 0 697 408 B1**
(45) Veröffentlichungstag und Bekanntmachung des Hinweises auf die Patenterteilung: **23.01.2002**
(21) Anmeldenummer: 95112583.0
(22) Anmeldetag: 10.08.1995
(51) Int. Cl.: C07D 263/20, A61K 31/42, C07D 413/10, C07D 413/06, C07D 413/14

(54) **Substituierte 1-phenyl-oxazoliden-2-one Derivate und ihre Verwendung als Adhäsionsrezeptor-Antagonisten**
1 substituted-phenyl-oxazolidene-2-one derivatives and their use as adhesion-receptor antagonists
Dérivés d'oxazolidène-2-one-1-phenyl substitué et leur utilisation comme antagonistes du récepteur d'adhésion

(30) Priorität: 19.08.1994 DE 4429461
(43) Veröffentlichungstag der Anmeldung: 21.02.1996
(73) Patentinhaber: MERCK PATENT GmbH, 64293 Darmstadt (DE)
(72) Erfinder: Juraszyk, Horst, Dr., D-64342 Seeheim (DE); Gante, Joachim, Prof. Dr., D-64291 Darmstadt (DE); Wurziger, Hanns, Dr., D-64291 Darmstadt (DE); Bernotat-Danielowski, Sabine, Dr., D-61231 Bad Nauheim (DE); Melzer, Guido, Dr., D-65719 Hofheim/Ts. (DE)

(56) Entgegenhaltungen:
- EP-A- 0 417 044
- EP-A- 0 645 376
- DE-A- 4 405 633
- FR-A- 2 068 423

## Beschreibung

Die Erfindung betrifft Oxazolidinoncarbonsäure-Derivate der Formel I 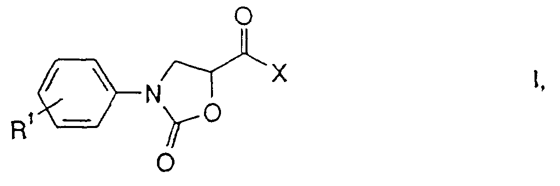 worin
- R¹: NO₂, NR⁶R⁷, CN, CONR⁶R⁷, CSNR⁶R⁷, C(=NH)SA, C(=NH)OA, C(=NH)SAr, C(=NH)NHOH, C(=NH)NR⁶R⁷, CH₂NR⁶R⁷, CH₂NHC(=NH)NR⁶R⁷, NHC(=NH)NR⁶R⁷, CH₂NHCO-alk-NR⁶R⁷, CH₂NHCO-Ph-E, CH₂NHCO-Ph-CH₂-NR⁶R⁷, CH₂NHCONH-Ph-E oder D,
- X: OH, OA, Z, Z-Z', 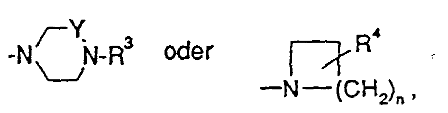
- D: 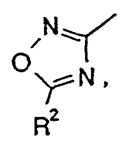
- E: -CN, -C(=NH)OA, -CSNH₂, -C(=NH)SA oder -C(=NH)NH₂,
- Y: CH₂, CHOR⁵ oder C=O,
- R2: H, A, Ar, OH, OA, CF₃, CCl₃, NR⁶R⁷, -alk-NR⁶R⁷, -alk(CH₂Ar)NR⁶R⁷, 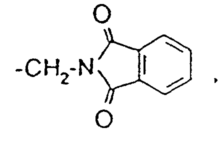
- R³: -(CH₂)ₘ-COOR⁵,
- R⁴: -(CH₂)ₚ-COOR⁵ oder -(CH₂)_{q}-O-(CH₂)ᵣ-COOR⁵,
- Z oder Z': jeweils unabhängig voneinander einen Aminosäurerest, ausgewählt aus einer Gruppe bestehend aus Ala, β-Ala, Arg, Asn, Asp, Gln, Glu, Gly, Leu, Lys, Orn, Phe, Pro, Sar, Ser, Thr, Tyr, Tyr (OMe), Val, C-Allyl-Gly, C-Propargyl-Gly, N-Benzyl-Gly, N-Phenethyl-Gly, N-Benzyl-β-Ala, N-Methyl-β-Ala und N-Phenethyl-β-Ala, wobei freie Amino- oder Carboxyl-Gruppen auch mit an sich bekannten konventionellen Schutzgruppen versehen sein können,
- R⁵, R⁶ und R7: jeweils unabhängig voneinander H oder A,
- m: 1,2 oder 3,
- n: 1,2,3 oder 4,
- p: 0,1 oder 2,
- q: 0 oder 1,
- r: 1 oder 2,
- A: Alkyl mit 1 bis 6 C-Atomen,
- -alk-: Alkylen mit 1 bis 6 C-Atomen,
- Ar: Phenyl oder Benzyl,
und
- Ph: Phenylen
bedeuten,
sowie ihre physiologisch unbedenklichen Salze und/oder Solvate.

Verbindungen mit einem ähnlichen Wirkungsprofil sind aus der EP-A1-0 381 033 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Diese Aufgabe wurde durch die Erfindung gelöst. Es wurde gefunden, daß die Verbindungen der Formel I sowie ihre Solvate und Salze bei guter Verträglichkeit wertvolle pharmakologische Eigenschaften besitzen. Insbesondere hemmen sie die Bindung von Fibrinogen, Fibronectin und des von Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Entzündungen und Arteriosklerose verwendet werden. Ferner haben die Verbindungen einen Effekt auf Tumorzellen, indem sie deren Metastasierung hemmen. Somit können sie auch als Anti-Tumor-Mittel eingesetzt werden.

Desweiteren eignen sich die Verbindungen zur Prophylaxe und zur Behandlung von osteolytischen Erkrankungen, insbesondere von Osteoporose und Restenose nach Angioplastie. Ferner haben sie antiangiogenetische Eigenschaften.

Die Verbindungen zeigen außerdem eine antimikrobielle Wirkung und können bei Behandlungen und Eingriffen eingesetzt werden, wo ein mikrobieller Befall verhindert werden muß.

Die Eigenschaften der Verbindungen können nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, 1962) nachweisen. Die Hemmung der Wechselwirkungen der β₃-Integrin-Rezeptoren mit geeigneten Liganden kann nach der Methode von J.W. Smith et al., J. Biol. Chem. 265, 12267-12271 (1990), nachgewiesen werden.

Gegenstand der Erfindung sind Verbindungen der angegebenen Formel I, ihre Salze und Solvate sowie ein Verfahren zur Herstellung dieser Verbindungen, dadurch gekennzeichnet, daß man
a) eine Verbindung der Formel I aus einem ihrer funktionellen Derivate durch Behandeln mit einem solvolysierenden oder hydrogenolysierenden Mittel in Freiheit setzt, oder daß man
b) eine Verbindung der Formel II 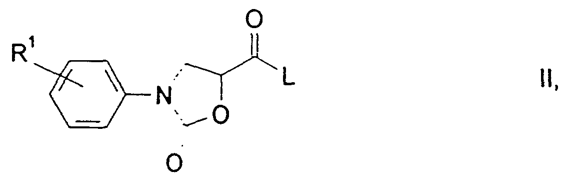 worin
   - R¹: die angegebene Bedeutung hat und
   - L: Cl, Br, OH oder eine reaktionsfähige veresterte OH-Gruppe bzw. leicht nucleophil substituierbare Abgangsgruppe bedeutet,
   mit einer Verbindung der Formel III

   H-X' III,

   worin X' Z, Z-Z', 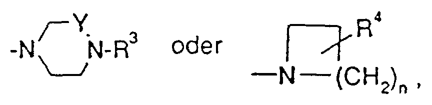 wobei Y, R³, R⁴ und n die angegebenen Bedeutungen besitzen, bedeutet, umsetzt, oder daß man
c) einen Rest X in einen anderen Rest X umwandelt, indem man
   - einen Ester der Formel I verseift, oder eine Carbonsäure der Formel I verestert, oder daß man
d) einen Rest R¹ in einen anderen Rest R¹ umwandelt, indem man
   - eine NO₂- und/oder CN-Gruppe katalytisch hydriert, oder
   - eine Nitrilgruppierung durch Umsetzung mit Ammoniak in eine C(=NH)-NH₂-Gruppe umwandelt, oder
   - eine Nitrilgruppe in eine Thiocarbamoylgruppe umwandelt, oder
   - eine Thiocarbamoylgruppe in eine Alkylsulfimidogruppe überführt, oder
   - eine Carbamoylgruppe in eine Alkylimidogruppe überführt, oder
   - eine Methylsulfimidogruppe in eine Amidin-Gruppe überführt, oder
   - eine Nitrilgruppe durch Umsetzung mit NH₂OH in eine C(=NH)-NHOH-Gruppe umwandelt, oder
   - eine NH₂-Gruppe in eine Guanidinylgruppe umwandelt, oder
   - eine C(= NH)-NHOH-Gruppe in eine Amidin-Gruppe umwandelt, oder
   - eine CH₂NH₂-Gruppe in eine Alkanoylaminomethyl-, eine CH₂NHC(=NH)NR⁶R⁷-, eine CH₂NHCO-Ph-C(=NH)NH₂, eine CH₂NHCO-Ph-CH₂NR⁶R⁷- oder eine CH₂NHCONH-Ph-E-Gruppe umwandelt, oder
   - eine 1,2,4-Oxadiazol- bzw. 1,2,4-Oxadiazolinon-Gruppe in eine Amidin-Gruppe umwandelt,
e) oder daß man eine Verbindung der Formel IV 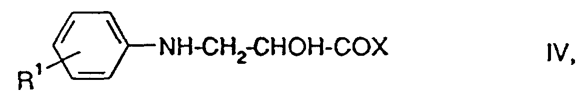 worin
   R¹ und X die angegebenen Bedeutungen haben, mit einem reaktionsfähigen Derivat der Kohlensäure umsetzt,
   und/oder daß man
f) eine Verbindung der Formel V 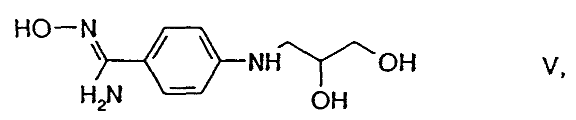 mit 2 Äquivalenten eines reaktionsfähigen Kohlensäurederivates umsetzt und anschließend oxidiert, oder daß man
g) eine Verbindung der Formel I durch Behandeln mit einer Säure oder einer Base in eines ihrer Salze überführt.

Die vor- und nachstehend aufgeführten Abkürzungen von Aminosäureresten stehen für die Reste folgender Aminosäuren:
- Ala: Alanin
- β-Ala: β-Alanin
- Arg: Arginin
- Asn: Asparagin
- Asp: Asparaginsäure
- Asp (O But): Asparaginsäure-β-butylester
- Gln: Glutamin
- Glu: Glutaminsäure
- Gly: Glycin
- Leu: Leucin
- Lys: Lysin
- Orn: Ornithin
- Phe: Phenylalanin
- Pro: Prolin
- Sar: Sarkosin (N-Methylglycin)
- Ser: Serin
- Thr: Threonin
- Tyr: Tyrosin
- Tyr (OMe): 2-Amino-3-p-methoxyphenylpropionsäure
- Val: Valin.

Ferner bedeuten nachstehend:
- BOC: tert-Butoxycarbonyl
- CBZ: Benzyloxycarbonyl
- DCCl: Dicyclohexylcarbodiimid
- DMF: Dimethylformamid
- EDCI: N-Ethyl-N'-(3-dimethylaminopropyl)-carbodiimidhydrochlorid
- Et: Ethyl
- Me: Methyl
- OMe: Methylester
- OEt: Ethylester
- TFA: Trifluoressigsäure.

Vor- und nachstehend haben die Reste R¹ und X die bei der Formel I angegebene Bedeutung. Sofern eine Verbindung der Formel I ein chirales Zentrum besitzt, kann sie in mehreren enantiomeren Formen auftreten. Alle diese Formen und deren Gemische, insbesondere Racemate, werden von der Erfindung mit eingeschlossen.

In den vor- und nachstehenden Formeln hat die Gruppe A 1-6, vorzugsweise 1, 2, 3 oder 4 C-Atome. Im einzelnen bedeutet A vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2- oder 3-Methylpentyl.

X ist vorzugsweise -OH, -OCH₃, -O-CH₂-CH₃, 4-Carboxypiperidino, 4-Carboxyalkylpiperidino, 4-Carboxyalkoxypiperidino sowie die entsprechenden Alkylestergruppen der genannten Reste, 4-Alkoxycarbonylpiperidino, 4-Carboxymethylpiperazino, 4-Carboxyethylpiperazino oder steht besonders bevorzugt für einen Aminosäure- oder einen Dipeptidrest, der über eine Amidbindung an die Carbonylgruppe gebunden ist. Sofern X einen Aminosäure- oder Dipeptidrest bedeutet, sind die folgenden besonders bevorzugt: Ala, β-Ala, Gly, Arg sowie β-Ala-Asp, Phe, N-Phenethylglycin, N-Phenethyl-β-Alanin oder Sar.

Der C-terminale Aminosäurerest kann dabei ebenfalls mit einer konventionellen Schutzgruppe verbunden sein. Insbesonders kommt eine Veresterung in Frage.

Die Gruppe R¹ steht vorzugsweise für -NH₂, -C(=NH)-NH₂, -CH₂-NH₂, -CH₂-NH-CO-alk-NH₂, -CH₂-NH-CO-Ph-C(=NH)-NH₂, -CH₂-NH-CO-Alk-C(=NH)-NH₂, -CH₂-NH-CO-Ph-CH₂-NH₂, NO₂ oder CN. Femer bedeutet R¹ außerdem bevorzugt -C(=NH)-S-A, -CSNH₂, -C(=NH)-NHOH oder

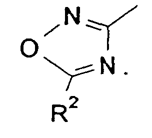

Der Rest Ar steht für unsubstituiertes Benzyl oder Phenyl.

Die Parameter m und n sind bevorzugt 1, ferner aber auch vorzugsweise 2 oder 3. Die Variable p ist vorzugsweise 0 oder 1, während q und r vorzugsweise 1 sind.

Unter den Verbindungen der Formel I sind diejenigen bevorzugt, in denen mindestens einer der angegebenen Reste, Gruppen und/oder Parameter eine der angegebenen bevorzugten Bedeutungen hat. Einige Gruppen von bevorzugten Verbindungen sind diejenigen der Formeln la bis If, die der Formel I entsprechen, worin jedoch
- in la: R¹ C(=NH)NH₂ und X OH oder OA bedeutet;
- in Ib: R¹ C(=NH)NH₂ und X 4-Carboxypiperidino, 4-Carboxyalkylpiperidino oder 4-Carboxyalkoxypiperidino bedeutet;
- in Ic: R¹ C(=NH)NH₂ und X β-Ala, Asp, Tyr, Tyr (OMe), N-Phenethyl-β-Ala oder Phe bedeutet, sowie die entsprechenden veresterten Derivate;
- in ld: R¹ (C=NH)NH₂ und X 4-Alkoxycarbonylpiperidino, 4-Alkoxycarbonylpiperazino, 4-Alkoxycarbonylalkylpiperidino, 4-Alkoxycarbonylalkylpiperazino oder 4-Alkoxycarbonylalkoxypiperidino bedeutet;
- in le: R¹ (C=NH)NH₂ und X 4-Carboxypiperazino oder 4-Carboxyalkylpiperazino bedeutet;
- in If: R¹ (C=NH)NHOH und X einen der unter la bis le genannten Reste bedeutet.

Desweiteren werden alle diejenigen Verbindungen von der Erfindung eingeschlossen, die über eine NH₂-Gruppe verfügen, wobei diese NH₂-Gruppe jedoch mit einer an sich bekannten Schutzgruppe versehen ist.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; femer J. March, Adv. Org. Chem., 3rd Ed. (1985), J. Wiley & Sons) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitro-phenyl (DNP)), Aralkoxymethyl- (z.B. Benzyloxymethyl (BOM)) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische; beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCI in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B.gut an 5-10%igem Pd-C in Methanol bei 20-30°.

Ferner kann z.B. eine hydrogenolytische Umwandlung einer 1,2,4-Oxadiazolin-5-on-3-yl- bzw. einer 5-Alkyl-1,2,4-Oxadiazol-3-yl-Gruppe in eine Amidin-Gruppe durch katalytische Hydrierung vorgenommen werden.

Verbindungen der Formel I können auch bevorzugt durch Reaktion eines Oxazolidinones der Formel II mit einer Verbindung der Formel III erhalten werden. Dabei bedient man sich zweckmäßig der an sich bekannten Methoden der nucleophilen Substitution und/oder der N-Alkylierung von Aminen bzw. den Reaktionen zur Amidbildung.

Die Abgangsgruppe L in der Formel II bedeutet vorzugsweise Cl, Br, oder OH bzw. eine daraus ableitbare Gruppe wie z.B. die Trifluormethansulfonyloxy-, Toluolsulfonyloxy- oder Methansulfonyloxygruppe.

Die Reaktion gelingt vorzugsweise in Gegenwart einer zusätzlichen Base, z.B. eines Alkali- oder Erdalkalimetall-hydroxids oder -carbonats wie Natrium-, Kalium- oder Calciumhydroxid, Natrium-, Kalium- oder Calciumcarbonat, in einem inerten Lösungsmittel z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 200, vorzugsweise zwischen 0 und 120°. Der Zusatz eines lodids wie Kaliumiodid kann den Ablauf der Reaktion begünstigen.

Die Ausgangsstoffe der Formel II sind in der Regel bekannt oder können in Analogie zu bekannten Verbindungen hergestellt werden. Ihre Herstellung ist beispielsweise in DE 37 23 797 (EP 300272) beschrieben. Sie können z.B. hergestellt werden durch Reaktion eines entsprechend substituierten Anilins mit Allylchlorid, anschließende Umwandlung der Doppelbindung in ein Diol, Umsetzung mit einem reaktionsfähigen Kohlensäurederivat, wie z.B. Phosgen, N,N-Carbonyldiimidazol, Kohlensäuredialkylester oder Diphosgen, Oxidation des Produktes zur 5-Oxazolidinoncarbonsäure und ggf. weitere Aktivierung durch Derivatisierung der Säuregruppe.

Es ist möglich in einer Verbindung der Formel II einen Rest L in einen anderen Rest L umzuwandeln, z.B. dadurch, daß man eine OH-Gruppe (Y = OH) mit SOCl₂, SOBr₂, Methansulfonylchlorid oder p-Toluolsulfonylchlorid umsetzt.

Die Verbindungen der Formel III sind in der Regel bekannt und kommerziell erhältlich.

Die Umsetzung der Oxazolidinone der Formel II mit den Verbindungen der Formel III erfolgt in an sich bekannten Weise, bevorzugt in einem protischen oder aprotischen polaren inerten Lösungsmittel bei Temperaturen zwischen 20° und dem Siedepunkt des Lösemittels. Die Reaktionszeiten betragen 10 Min. bis 24 Std., vorzugsweise 2 Std. bis 10 Std.

Als Lösungsmittel eignen sich insbesondere auch Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander. Besonders geeignet ist N-Methylpyrrolidon.

Derivate mit freier primärer oder sekundärer Aminogruppe werden zweckmäßig in geschützter Form umgesetzt. Als Schutzgruppen kommen die zuvor genannten in Frage.

Es ist ferner möglich, eine Verbindung der Formel I zu erhalten, indem man einen Rest X in einen anderen Rest X umwandelt. Beispielsweise kann man eine freie Säuregruppe (X = OH) verestern (X = OA) oder über eine Peptidbindung mit einer Aminosäure oder einem Dipeptid verknüpfen. Außerdem kann man z.B. auch eine Säure in ein Amid umwandeln.

Weiterhin ist es möglich eine Verbindung der Formel I zu erhalten, indem man einen Rest R¹ in einer Verbindung der Formel I in einen anderen Rest R¹ umwandelt.

Insbesondere kann man Cyangruppen zu Aminomethylgruppen reduzieren oder in Amidinogruppen umwandeln, Benzylgruppen hydrogenolytisch entfernen, Aminomethylgruppen in Guanidinomethylgruppen überführen oder Nitrilgruppen in Thiocarbamoylgruppen überführen.

Eine Reduktion von Cyangruppen zu Aminomethylgruppen gelingt zweckmäßigerweise durch katalytische Hydrierung, z.B. an Raney-Nickel bei Temperaturen zwischen 0 und 100°, vorzugsweise 10 und 30°, und Drucken zwischen 1 und 200 bar, vorzugsweise bei Normaldruck, in einem inerten Lösungsmittel, z.B. einem niederen Alkohol wie Methanol oder Ethanol, zweckmäßig in Gegenwart von Ammoniak. Arbeitet man z.B. bei etwa 20° und 1 bar, so bleiben im Ausgangsmaterial vorhandene Benzylester- oder N-Benzylgruppen erhalten. Will man diese hydrogenolytisch spalten, so verwendet man zweckmäßig einen Edelmetallkatalysator, vorzugsweise Pd-Kohle, wobei man der Lösung eine Säure wie Essigsäure sowie auch Wasser zusetzen kann.

Zur Herstellung von Verbindungen der Formel I, worin R¹ eine Guanidinophenylgruppe bedeutet, kann man eine entsprechende Aminophenylverbindung mit einem amidinierenden Mittel behandeln. Als amidinierendes Mittel ist 1-Amidino-3,5-dimethylpyrazol bevorzugt, das insbesondere in Form seines Nitrats eingesetzt wird. Man arbeitet zweckmäßig unter Zusatz einer Base wie Triethylamin oder Ethyl-diisopropylamin in einem inerten Lösungsmittel oder Lösungsmittelgemisch, z.B. Wasser/Dioxan bei Temperaturen zwischen 0 und 120°, vorzugsweise 60 und 120°.

Zur Herstellung eines Amidins der Formel I kann man an ein Nitril der Formel I Ammoniak anlagern. Die Anlagerung erfolgt bevorzugt mehrstufig, indem man in an sich bekannter Weise a) das Nitril mit H₂S in ein Thioamid umwandelt, das mit einem Alkylierungsmittel, z.B. CH₃I, in den entsprechenden S-Alkyl-imidothioester übergeführt wird, welcher seinerseits mit NH₃ zum Amidin reagiert, b) das Nitril mit einem Alkohol, z.B. Ethanol in Gegenwart von HCI in den entsprechenden Imidoester umwandelt und diesen mit Ammoniak behandelt, oder c) das Nitril mit Lithium-bis-(trimethylsilyl)-amid umsetzt und das Produkt anschließend hydrolysiert.

Analog sind die entsprechenden N-Hydroxy-amidine der Formel I aus den Nitrilen erhältlich, wenn man nach a) oder b), aber mit Hydroxylamin anstelle von Ammoniak arbeitet.

Weiterhin kann man N-Hydroxy-amidine durch Umsetzung mit aliphatischen Carbonsäurechloriden in 1,2,4-Oxadiazole bzw. 1,2,4-Oxadiazolinone überführen und diese dann durch katalytische Hydrierung, z.B. an Raney-Ni, Pd/C oder PtO₂, vorzugsweise in MeOH, Dioxan, Eisessig, Eisessig/Essigsäureanhydrid oder DMF, in Amidine überführen.

Ferner kann man eine Verbindung der Formel I erhalten, indem man eine Verbindung der Formel IV mit einem reaktionsfähigen Derivat der Kohlensäure umsetzt. Reaktionsfähige Abkömmlinge der Kohlensäure können beispielsweise die zuvor genannten sein, wobei Phosgen und Diphosgen sowie N,N-Carbonyldiimidazol besonders bevorzugt sind. Als Kohlensäurederivate eignen sich ferner besonders Dialkylcarbonate wie Diethylcarbonat, ferner auch Chlorameisensäurealkylester wie Ethylchlorformiat. Bevorzugt dient das Kohlensäurederivat, das zweckmäßig im Überschuß eingesetzt wird, auch als Lösungs- bzw. Suspensionsmittel. Es kann aber auch eines der angegebenen Lösungsmittel anwesend sein, sofern es bei dieser Umsetzung inert ist. Weiterhin empfiehlt sich der Zusatz einer Base, insbesondere eines Alkalimetallalkoholats wie Kalium-tert.-butylat. Man arbeitet zweckmäßig bei Reaktionstemperatur zwischen 0 und 150°, vorzugsweise zwischen 70 und 120°.

Die Ausgangsstoffe der Formel IV sind in der Regel neu. Sie sind z.B. erhältlich, indem man ein am Aromaten substituiertes Anilin mit einer α-Hydroxy-β-halogen-carbonsäure umsetzt.

Es ist außerdem möglich, eine Verbindung der Formel I zu erhalten, indem man ein Diol der Formel V mit einem Überschuß eines reaktionsfähigen Kohlensäurederivates umsetzt, und zwar vorzugsweise unter den zuvor genannten Bedingungen. Während die Kohlensäurederivate kommerziell erhältlich sind, können die Verbindungen der Formel V z.B. durch Umsetzung von Allylchlorid mit p-Aminobenzonitril, Umwandlung der Doppelbindung in eine Dihydroxygruppierung und Umsetzung mit Hydroxylamin erhalten werden.

Eine Base der Formel l kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Es ist auch möglich, Carbonsäuren der Formel 1 durch Umsetzung mit entsprechenden Basen in ihre Metall- oder Ammoniumsalze umzuwandeln, z.B. ihre Natrium-, Kalium- oder Calciumsalze.

Die Verbindungen der Formel I können ein oder mehrere chirale Zentren enthalten und können daher in racemischer oder in optisch-aktiver Form vorliegen. Erhaltene Racemate können nach an sich bekannten Methoden mechanisch oder chemisch in die Enantiomeren getrennt werden.

Vorzugsweise werden aus dem racemischen Gemisch durch Umsetzung mit einem optischaktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optischaktiven Camphersulfonsäuren wie β-Camphersulfonsäure.

Vorteilhaft ist auch eine Enantiomerentrennung mit Hilfe einer mit einem optisch aktiven Trennmittel (z.B. Dinitrobenzoyl-phenyl-glycin) gefüllten Säule; als Laufmittel eignet sich z.B. ein Gemisch Hexan/lsopropanol/ Acetonitril.

Natürlich ist es auch möglich, optischaktive Verbindungen der Formel I nach den oben beschriebenen Methoden zu erhalten, indem man Ausgangsstofle (z.B. solche der Formel II) verwendet, die bereits optischaktiv sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Pharmaka, insbesondere aber in Analogie zu den in der EP-A-459256 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation. Sofern Molmassen angegeben werden, so werden herkömmliche massenspektroskopische Werte mit "M": und "fast atom bombardment"-Werte, sogenannte FAB-Werte, mit "M + 1" bezeichnet.

### Beispiel 1

Zu einer Lösung von 3,6 g Na-dihydrogenphosphat-Dihydrat, 3,9 g Nametaperiodat und 36 mg RuCl₃ in 23 ml Wasser/2,3 ml Dichlormethan werden bei 16° 1 g 4-(2-Oxo-5-hydroxymethyloxazolidin-3-yl)-benzonitril [erhältlich nach EP 300272] gelöst in 12 ml Acetonitril zugegeben. Das Reaktionsgemisch wird 12 Std. bei Raumtemperatur gerührt, filtriert und wie üblich aufgearbeitet. Nach Einengen zur Trockne im Vakuum erhält man 3-(4-Cyanphenyl)-2-oxo-5-oxazolidincarbonsäure, F. 204°.

### Beispiel 2

27,9 g 3-(4-Cyanphenyl)-2-oxo-5-oxazolidincarbonsäure [erhältlich gemäß Bsp. 1] und 26,4 g Hydroxylammoniumchlorid werden in einer Mischung aus 750 ml Methanol und 30 ml Wasser in Gegenwart von 53,4 g Natriumcarbonat 5 Std. gekocht. Der entstandene Niederschlag wird abgesaugt, mit Methanol gewaschen und getrocknet. Man erhält nach Behandlung mit Salzsäure 3-[4-(Amino-(hydroxylimino)methyl)-phenyl]-2-oxo-5-oxazolidincarbonsäure, Hydrochlorid, F. 205-208°.

### Beispiel 3

Man löst 13,9 g 3-[4-(Amino-(hydroxylimino)methyl)-phenyl]-2-oxo-5-oxazolidincarbonsäure [erhältlich gemäß Bsp. 2] in 210 ml Eisessig, fügt 11,3 g Chlorameisensäure-trichlormethylester hinzu und kocht 3 Std. Anschließend kühlt man auf Raumtemperatur ab und arbeitet wie üblich auf. Man erhält 3-[4-(5-Oxo-1,2,4-oxadiazolin-3-yl)-phenyl]-2-oxo-5-oxazolidincarbonsäure, F. 250-253°.

### Beispiel 4

Zu einer Lösung von 2,91 g 3-[4-(5-Oxo-1,2,4-oxadiazolin-3-yl)-phenyl]-2-oxo-5-oxazolidincarbonsäure [erhältlich gemäß Bsp. 3], 2,77 g Piperidin-4-carbonsäure-tert.-butylester, 2,02 g 1-Hydroxybenzotriazol und 2,86 g N-(3-Dimethylaminopropyl)-N'-ethylcarbodiimid, Hydrochlorid in 20 ml DMF gibt man 3,03 g N-Methylmorpholin hinzu und rührt 5 Std. bei Raumtemperatur. Anschließend fügt man unter intensivem Rühren 200 ml Wasser tropfenweise hinzu, trennt den entstandenen kristallinen Niederschlag ab, nimmt diesen in 90 ml Dichlormethan auf und trocknet über Na₂SO₄. Man engt die Lösung im Vakuum ein, verreibt das erhaltene Öl mit Diethylether und erhält 1-{3-[4-(5-Oxo-1,2,4-oxadiazolin-3-yl)-phenyl]-2-oxo-5-oxazolidinylcarbonyl}-piperidin-4-carbonsäure-tert.-butylester, F. 171-175°.

Analog erhält man durch Umsetzung von 3-[4-(5-Oxo-1,2,4-oxadiazolin-3-yl)-phenyl]-2-oxo-5-oxazolidincarbonsäure
mit 2-(Piperidin-4-yl)-essigsäure-tert.-butylester:
   2-{1-[3-(4-(5-Oxo-1,2,4-oxadiazolin-3-yl)-phenyl)-2-oxo-5-oxazolidinylcarbonyl]-piperidin-4-yl}-essigsäure-tert.-butylester;
mit 2-(Piperidin-4-yl)-essigsäureethylester:
   2-{1-[3-(4-(5-Oxo-1,2,4-oxadiazolin-3-yl)-phenyl)-2-oxo-5-oxazolidinylcarbonyl]-piperidin-4-yl}-essigsäureethylester, F. 178-181°;
mit 2-(Piperidin-4-yl-oxy)-essigsäure-tert.-butylester:
   2-{1-[3-(4-(5-Oxo-1,2,4-oxadiazolin-3-yl)-phenyl)-2-oxo-5-oxazolidinylcarbonyl]-piperidin-4-yl-oxy}-essigsäure-tert.-butylester, F. 180-181°;
mit 2-(Piperidin-3-yl-oxy)-essigsäure-tert.-butylester:
   2-{1-[3-(4-(5-Oxo-1,2,4-oxadiazolin-3-yl)-phenyl)-2-oxo-5-oxazolidinylcarbonyl]-piperidin-3-yl-oxy}-essigsäure-tert.-butylester, F. 92-95°;
mit 2-(2-Oxo-piperazino)-essigsäure-tert.-butylester:
   2-{4-[3(4-(5-Oxo-1,2,4-oxadiazolin-3-yl)-phenyl)-2-oxo-5-oxazolidinylcarbonyl]-2-oxo-piperazino}-essigsäure-tert.-butylester, F. 155-157°;
mit (2R)-2-Aminopropionsäure-tert.-butylester (H-Ala-OBut):
   (2R)-2-{3-[4-(5-Oxo-1,2,4-oxadiazolin-3-yl)-phenyl]-2-oxo-5-oxazolidinylcarbonylamino}-propionsäure-tert.-butylester, F. 105-107°;
mit (2R)-2-Aminopropionsäure-methylester (H-Ala-OMe):
   (2R)-2-{3-[4-(5-Oxo-1,2,4-oxadiazolin-3-yl)-phenyl]-2-oxo-5-oxazolidinylcarbonylamino}-propionsäuremethylester, F. 199-201°;
mit (2R)-2-Aminobernsteinsäure-di-tert.-butylester (H-Asp(OBut)-OBut):
   (2R)-2-{3-[4-(5-Oxo-1,2,4-oxadiazolin-3-yl)-phenyl]-2-oxo-5-oxazolidinylcarbonylamino}-bernsteinsäure-di-tert.-butylester, F. 175-176°;
mit 3-Aminopropionsäure-tert.-butylesler (H-βAla-OBut):
   3-{3-[4-(5-Oxo-1,2,4-oxadiazolin-3-yl)-phenyl]-2-oxo-5-oxazolidinylcarbonylamino)-propionsäure-tert.-butylester, F. 143-146°;
mit 3-Aminopropionsäure-tert.-methylester (H-βAla-OMe):
   3-{3-[4-(5-Oxo-1,2,4-oxadiazolin-3-yl)-phenyl]-2-oxo-5-oxazolidinylcarbonylamino}-propionsäuremethylester, F. 230-232°;
mit (2R)-2-Amino-3-(4-hydroxyphenyl)-propionsäure-tert.-butylester (H-Tyr-OBut):
   (2R)-2-{3-[4-(5-Oxo-1,2,4-oxadiazolin-3-yl)-phenyl]-2-oxo-5-oxazolidinylcarbonylamino}-3-(4-hydroxyphenyl)-propionsäure-tert.-butylester;
mit (2R)-2-Amino-3-(4-melhoxyphenyl)-propionsäure-tert.-butylester (H-Tyr(OMe)-OBut):
   (2R)-2-{3-[4-(5-Oxo-1,2,4-oxadiazolin-3-yl)-phenyl]-2-oxo-5-oxazolidinylcarbonylamino}-3-(4-methoxyphenyl)-propionsäure-tert.-butylester;
mit 3-N-Phenethyl-aminopropionsäure-tert.-butylester:
   3-{3-[4-(5-Oxo-1,2,4-oxadiazolin-3-yl)-phenyl]-2-oxo-5-oxazolidinylcarbonyl-(N-phenethyl)-amino}-propionsäure-tert.-butylester;
mit 2-Piperazino-essigsäure-benzylester:
   2-{1-[3-(4-(5-Oxo-1,2,4-oxadiazolin-3-yl)-phenyl)-2-oxo-5-oxazolidinylcarbonyl]-piperazin-4-yl}-essigsäurebenzylester, F. 165-170°;
mit 3-Piperazino-propionsäure-benzylester:
   3-{1-[3-(4-(5-Oxo-1,2,4-oxadiazolin-3-yl)-phenyl)-2-oxo-5-oxazolidinylcarbonyl]-piperazin-4-yl}-propionsäurebenzylester, F. 150-153°.

### Beispiel 5

1,37 g 1-{3-[4-(5-Oxo-1,2,4-oxadiazolin-3-yl)-phenyl]-2-oxo-5-oxazolidinylcarbonyl}-piperidin-4-carbonsäure-tert.-butylester werden in 50 ml Methanol gelöst und an Raney-Nickel hydriert. Anschließend wird die Reaktionsmischung filtriert und das Fittrat im Vakuum eingeengt. Das erhaltene Produkt wird mit 20 ml Essigsäureethylester in der Wärme behandelt und das Produkt nach Abkühlen abgesaugt. Man erhält 1-[3-(4-Amidinophenyl)-2-oxo-5-oxazolidinylcarbonyl}-piperidin-4-yl-carbonsäure-tert.-butylester, F. 160°.

Analog erhält man durch reduktive Spaltung der 5-Oxo-1,2,4-oxadiazolin-Gruppe
aus 2-{1-[3-(4-(5-Oxo-1,2,4-oxadiazolin-3-yl)-phenyl)-2-oxo-5-oxazolidinylcarbonyl]-piperidin-4-yl}-essigsäure-tert.-butylester:
   2-{1-[3-(4-Amidinophenyl)-2-oxo-5-oxazolidinylcarbonyl]-piperidin-4-yl-}-essigsäure-tert.-butylester;
aus 2-{1-[3-(4-(5-Oxo-1,2,4-oxadiazolin-3-yl)-phenyl)-2-oxo-5-oxazolidinylcarbonyl]-piperidin-4-yl}-essigsäureethylester:
   2-{1-[3-(4-Amidinophenyl)-2-oxo-5-oxazolidinylcarbonyl]-piperidin-4-yl-}-essigsäureethylester, F. 210-211°;
aus 2-{1-[3-(4-(5-Oxo-1,2,4-oxadiazolin-3-yl)-phenyl)-2-oxo-5-oxazolidinylcarbonyl]-piperidin-4-yl-oxy}-essigsäure-tert.-butylester:
   2-{1-[3-(4-Amidinophenyl)-2-oxo-5-oxazolidinylcarbonyl]-piperidin-4-yl-oxy}-essigsäure-tert.-butylester, F. 100°;
aus 2-{1-[3-(4-(5-Oxo-1,2,4-oxadiazolin-3-yl)-phenyl)-2-oxo-5-oxazolidinylcarbonyl]-piperidin-3-yl-oxy}-essigsäure-tert.-butylester:
   2-{1-[3-(4-Amidinophenyl)-2-oxo-5-oxazolidinylcarbonyl]-piperidin-3-yl-oxy}-essigsäure-tert.-butylester, F. 139-144°;
aus 2-{4-[3-(4-(5-Oxo-1,2,4-oxadiazolin-3-yl)-phenyl)-2-oxo-5-oxazolidinylcarbonyl]-2-oxo-piperazino}-essigsäure-tert.-butylester:
   2-{4-[3-(4-Amidinophenyl)-2-oxo-5-oxazolidinylcarbonyl]-2-oxopiperazino}-essigsäure-tert.-butylester, F. 165-167°;
aus (2R)-2-{3-[4-(5-Oxo-1,2,4-oxadiazolin-3-yl)-phenyf]-2-oxo-5-oxazolidinylcarbonylamino}-propionsäure-tert.-butylester:
   (2R)-2-[3-(4-Amidinophenyl)-2-oxo-5-oxazolidinylcarbonylamino]-propionsäure-tert.-butylester, F. 173-175°;
aus (2R)-2-(3-[4-(5-Oxo-1,2,4-oxadiazolin-3-yl)-phenyl]-2-oxo-5-oxazolidinylcarbonylamino}-propionsäure-methylester:
   (2R)-2-[3-(4-Amidinophenyl)-2-oxo-5-oxazolidinylcarbonylamino]-propionsäuremethylester, Acetat, F. 190-192°;
aus (2R)-2-{3-[4-(5-Oxo-1,2,4-oxadiazolin-3-yl)-phenyl]-2-oxo-5-oxazolidinylcarbonylamino}-bernsteinsäure-di-tert.-butylester:
   (2R)-2-[3-(4-Amidinophenyl)-2-oxo-5-oxazolidinylcarbonylamino]-bernsteinsäure-di-tert.-butylester, Acetat, F. 242°;
aus 3-{3-[4-(5-Oxo-1,2,4-oxadiazolin-3-yl)-phenyl]-2-oxo-5-oxazolidinyl-carbonylamino}-propionsäure-tert.-butylester:
   3-[3-(4-Amidinophenyl)-2-oxo-5-oxazolidinylcarbonylamino]-propionsäure-tert.-butylester, F. 164-167°;
aus 3-{3-[4-(5-Oxo-1,2,4-oxadiazolin-3-yl)-phenyl]-2-oxo-5-oxazolidinylcarbonylamino}-propionsäure-methylester:
   3-[3-(4-Amidinophenyl)-2-oxo-5-oxazolidinylcarbonylamino]-propionsäuremethylester, Acetat, F. 207-209°;
aus (2R)-2-{3-[4-(5-Oxo-1,2,4-oxadiazolin-3-yl)-phenyl]-2-oxo-5-oxazolidinylcarbonylamino}-3-(4-hydroxyphenyl)-propionsäure-tert.-butylester:
   (2R)-2-[3-(4-Amidinophenyl)-2-oxo-5-oxazolidinylcarbonylamino]-3-(4-hydroxyphenyl)-propionsäure-tert.-butylester;
aus (2R)-2-{3-[4-(5-Oxo-1,2,4-oxadiazolin-3-yl)-phenyl]-2-oxo-5-oxazolidinylcarbonylamino}-3-(4-methoxyphenyl)-propionsäure-tert.-butylester:
   (2R)-2-[3-(4-Amidinophenyl)-2-oxo-5-oxazolidinylcarbonylamino]-3-(4-methoxyphenyl)-propionsäure-tert.-butylester;
aus 3-{3-[4-(5-Oxo-1,2,4-oxadiazolin-3-yl)-phenyl]-2-oxo-5-oxazolidinylcarbonyl-(N-phenethyl)amino}-propionsäure-tert.-butylester:
   3-[3-(4-Amidinophenyl)-2-oxo-5-oxazolidinylcarbonyl-(N-phenethyl)-amino]-propionsäure-tert.-butylester,
aus 2-{1-[3-(4-(5-Oxo-1,2,4-oxadiazolin-3-yl)-phenyl)-2-oxo-5-oxazolidinylcarbonyl]-piperazin-4-yl}-essigsäure-benzylester:
   2-{1-[3-(4-Amidinophenyl)-2-oxo-5-oxazolidinylcarbonyl]-piperazin-4-yl}-essigsäurebenzylester;
aus 3-{1-[3-(4-(5-Oxo-1,2,4-oxadiazolin-3-yl)-phenyl)-2-oxo-5-oxazolidinylcarbonyl]-piperazin-4-yl}-propionsäure-benzylester:
   3-{1-[3-(4-Amidinophenyl)-2-oxo-5-oxazolidinylcarbonyl]-piperazin-4-yl}propionsäurebenzylester.

### Beispiel 6

0,41 g 1-[3-(4-Amidinophenyl)-2-oxo-5-oxazolidinylcarbonyl}-piperidin-4-yl-carbonsäure-tert.-butylester werden in 40 ml etherischer HCI-Lösung 2 Std. bei Raumtemperatur gerührt. Der entstandene Niederschlag wird abgesaugt und mit 20 ml Ether gewaschen. Anschließend wird das Produkt mit 5 ml Acetonitril 10 Min. bei 60° behandelt, nach Abkühlen auf Raumtemperatur abgesaugt und mit wenig Acetonitril gewaschen. Man erhält 1-[3-(4-Amidinophenyl)-2-oxo-5-oxazolidinylcarbonyl}-piperidin-4-carbonsäure, Hydrochlorid, F. 184°(Zers.).

Analog erhält man durch Verseifung der entsprechenden Ester
aus 2-{1-[3-(4-Amidinophenyl)-2-oxo-5-oxazolidinylcarbonyl]-piperidin-4-yl}-essigsäure-tert.-butylester:
   2-{1-[3-(4-Amidinophenyl)-2-oxo-5-oxazolidinylcarbonyl]-piperidin-4-yl}-essigsäure, Hydrochlorid;
aus 2-{1-[3-(4-Amidinophenyl)-2-oxo-5-oxazolidinylcarbonyl]-piperidin-4-yl-oxy}-essigsäure-tert.-butylester:
   2-{1-[3-(4-Amidinophenyl)-2-oxo-5-oxazolidinylcarbonyl]-piperidin-4-yl-oxy}-essigsäure, Hydrochlorid, F. 170-175° (Zers.);
aus 2-{1-[3-(4-Amidinophenyl)-2-oxo-5-oxazolidinylcarbonyl]-piperidin-3-yl-oxy}-essigsäure-tert.-butylester:
   2-{1-(3-(4-Amidinophenyl)-2-oxo-5-oxazolidinylcarbonyl]-piperidin-3-yl-oxy}-essigsäure, Hydrochlorid, F. 127-129°;
aus 2-{4-[3-(4-Amidinophenyl)-2-oxo-5-oxazolidinylcarbonyl]-2-oxopiperazino}-essigsäure-tert.-butylester:
   2-{4-[3-(4-Amidinophenyl)-2-oxo-5-oxazolidinylcarbonyl]-2-oxopiperazino}-essigsäure, Hydrochlorid, F. 63-70°;
aus (2R)-2-[3-(4-Amidinophenyl)-2-oxo-5-oxazolidinylcarbonylamino]-propionsäure-tert.-butylester:
   (2R)-2-[3-(4-Amidinophenyl)-2-oxo-5-oxazolidinylcarbonylamino]-propionsäure, Hydrochlorid, F. 190-194°;
aus (2R)-2-[3-(4-Amidinophenyl)-2-oxo-5-oxazolidinylcarbonylamino]-bernsteinsäure-di-tert.-butylester:
   (2R)-2-[3-(4-Amidinophenyl)-2-oxo-5-oxazolidinylcarbonylamino]-bernsteinsäure, Hydrochlorid, F. 308-310° Zers.);
aus 3-[3-(4-Amidinophenyl)-2-oxo-5-oxazolidinylcarbonylamino]-propionsäure-tert. -butylester:
   3-[3-(4-Amidinophenyl)-2-oxo-5-oxazolidinylcarbonylamino]-propionsäure, Hydrochlorid, F. 243-244° (Zers.);
aus (2R)-2-[3-(4-Amidinophenyl)-2-oxo-5-oxazolidinylcarbonylamino]-3-(4-hydroxyphenyl)-propionsäure-tert.-butylester:
   (2R)-2-[3-(4-Amidinophenyl)-2-oxo-5-oxazolidinylcarbonylamino]-3-(4-hydroxyphenyl)-propionsäure, Hydrochlorid;
aus (2R)-2-[3-(4-Amidinophenyl)-2-oxo-5-oxazolidinylcarbonylamino]-3-(4-methoxyphenyl)-propionsäure-tert.-butylester.
   (2R)-2-[3-(4-Amidinophenyl)-2-oxo-5-oxazolidinylcarbonylamino]-3-(4-methoxyphenyl)-propionsäure, Hydrochlorid;
aus (2R)-2-[3-(4-Amidinophenyl)-2-oxo-5-oxazolidinylcarbonyl-(N-phenethyl)-amino]-propionsäure-tert.-butylester:
   (2R)-2-[3-(4-Amidinophenyl)-2-oxo-5-oxazolidinylcarbonyl-(N-phenethyl)-amino]-propionsäure, Hydrochlorid;

### Beispiel 7

1,0 g 2-{1-[3-(4-Amidinophenyl)-2-oxo-5-oxazolidinylcarbonyl]-piperazin-4-yl}-essigsäure-benzylester werden in einer Mischung aus 100 ml Methanol und 50 ml DMF an Pd-Kohle bis zum Ende der Wasserstoffaufnahme katalytisch hydriert. Anschließend wird die Reaktionsmischung filtriert und wie üblich aufgearbeitet. Nach Verreiben des Rohproduktes mit Ether und Trocknung erhält 2-{1-[3-(4-Amidinophenyl)-2-oxo-5-oxazolidinylcarbonyl]-piperazin-4-yl}-essigsäure, F.220-225°(Zers.).

Analog erhält man
aus 3-{1-[3-(4-Amidinophenyl)-2-oxo-5-oxazolidinylcarbonyl]-piperazin-4-yl}-propionsäure-benzylester:
   3-{1-[3-(4-Amidinophenyl)-2-oxo-5-oxazolidinylcarbonyl)-piperazin-4-yl}-propionsäure, F. 255-258° (Zers.).

### Beispiel 8

Analog zu Beispiel 4 erhält man durch Umsetzung von Phenylalanin-tert.-butylester und 3-(4-Cyanphenyl)-2-oxo-5-oxazolidin-carbonsäure [erhältlich gemäß Bsp. 1] den (2R)-2-[3-(4-Cyanphenyl)-2-oxo-5-oxazolidinylcarbonylamino]-3-phenyl-propionsäure-tert.-butylester, F. 72°.

Analog erhält man durch Umsetzung von 3-(4-Cyanphenyl)-2-oxo-5-oxazolidin-carbonsäure
mit Piperidin-4-carbonsäurebenzylester:
   1-[3-(4-Cyanphenyl)-2-oxo-5-oxazolidinylcarbonyl]-piperidin-4-carbonsäurebenzylester, FAB (M+1): 434;
mit 2-(4-Piperidinyloxy)-essigsäure-tert.-butylester:
   2-{1-[3-(4-Cyanphenyl)-2-oxo-5-oxazolidinylcarbonyl]-4-piperidinyloxy}-essigsäure-tert.-butylester, F. 126-127°.

### Beispiel 9

2,3 g 3-(4-Cyanphenyl)-2-oxo-5-oxazolidincarbonsäurechlorid [F. 148-150°; erhältlich aus der Säure durch Umsetzung mit Oxalylchlorid] werden mit einem Equivalent 1-Piperazinylessigsäurebenzylester, Hydrochlorid in 100 ml Dichlormethan in Gegenwart von 5 ml Triethylamin bei Raumtemperatur umgesetzt. Nach üblicher Aufarbeitung erhält man 2-{1-[3-(4-Cyanphenyl)-2-oxo-5-oxazolidinyl-carbonyl]-4-piperazinyl}-essigsäurebenzylester, F. 131-132°.

### Beispiel 10

Man löst 2,6 g (2R)-2-[3-(4-Cyanphenyl)-2-oxo-5-oxazolidinylcarbonylamino]-3-phenylpropionsäure-tert.-butylester [erhältlich nach Bsp. 8] in einem Lösungsmittelgemisch bestehend aus 30 ml Pyridin und 5 ml Triethylamin und rührt 1,5 Std unter Eiskühlung, wobei während dieser Phase kontinuierlich H₂S-Gas eingeleitet wird. Anschließend rührt man die Reaktionsmischung noch 24 Std. bei Raumtemperatur. Nach Eindampfen und üblicher Aufarbeitung erhält man (2R)-2-[3-(4-Thiocarbamoyl-phenyl)-2-oxo-5-oxazolidinylcarbonylamino]-3-phenyl-propionsäure-tert.-butylester, F. 185-186°.

Analog erhält man
aus 1-[3-(4-Cyanphenyl)-2-oxo-5-oxazolidinylcarbonyl]-piperidin-4-carbonsäurebenzylester:
   1-[3-(4-Thiocarbamoyl-phenyl)-2-oxo-5-oxazolidinylcarbonyl]-piperidin-4-carbonsäurebenzylester, F. 167-169°;
aus 2-{1-[3-(4-Cyanphenyl)-2-oxo-5-oxazolidinylcarbonyl]-4-piperidinyloxy}-essigsäure-tert.-butylester:
   2-{1-[3-(4-Thiocarbamoyl-phenyl)-2-oxo-5-oxazolidinylcarbonyl]-4-piperidinyloxy}-essigsäure-tert.-butylester, F. 190-191°;
aus 2-{1-[3-(4-Cyanphenyl)2-oxo-5-oxazolidinyl-carbonyl]-4-piperazinyl}-essigsäurebenzylester:
   2-{1-[3-(4-Thiocarbamoyl-phenyl)-2-oxo-5-oxazolidinyl-carbonyl]-4-piperazinyl}-essigsäurebenzylester.

### Beispiel 11

0,92 g (2R)-2-[3-(4-Thiocarbamoyl-phenyl)-2-oxo-5-oxazolidinylcarbonylamino]-3-phenylpropionsäure-tert.-butylester [erhältlich nach Bsp. 10] werden in 15 ml Aceton gelöst und mit 1,75 ml Methyliodid versetzt. Die Lösung wird 2 Std. bei Raumtemperatur gerührt und wie üblich aufgearbeitet. Man erhält (2R)-2-[3-(4-lmino-(methylthio)-methylphenyl)-2-oxo-5-oxazolidinyl-carbonylamino]-3-phenylpropionsäure-tert.-butylester, Hydroiodid, F. 140°.

Analog erhält man
aus 1-[3-(4-Thiocarbamoyl-phenyl)-2-oxo-5-oxazolidinylcarbonyl]-piperidin-4-carbonsäure-benzylester:
   1-[3-(4-Imino-(methylthio)-methylphenyl)-2-oxo-5-oxazolidinylcarbonyl]-piperidin-4-carbonsäurebenzylester, Hydroiodid, F. 86-91°;
aus 2-{1-[3-(4-Thiocarbamoyl-phenyl)-2-oxo-5-oxazolidinylcarbonyl]-4-piperidinyloxy}-essigsäure-tert.-butylester:
   2-{1-[3-(4-Imino-(methylthio)-methylphenyl)-2-oxo-5-oxazolidinylcarbonyl]-4- piperidinyloxy}-essigsäure-tert.-butylester, F. 157°;
aus 2-{1-[3-(4-Thiocarbamoyl-phenyl)-2-oxo-5-oxazolidinyl-carbonyl]-4-piperazinyl}-essigsäure-benzylester:
   2-{1-[3-(4-Imino-(methylthio)-methylphenyl)-2-oxo-5-oxazolidinylcarbonyl]-4-piperazinyl}-essigsäurebenzylester.

### Beispiel 12

Man suspendiert 0,45 g (2R)-2-[3-(4-lmino-(methylthio)-methylphenyl)-2-oxo-5-oxazolidinyl-carbonylamino]-3-phenylpropionsäure-tert.-butylester [erhältlich nach Bsp. 11] in 5 ml Methanol, fügt 0,47 g Ammoniumacetat hinzu und rührt 24 Std bei Raumtemperatur. Anschließend versetzt man die Reaktionsmischung mit 10 ml Ether und trennt den entstandenen Niederschlag ab. Nach Eindampfen und üblicher Aufarbeitung sowie Behandlung mit Eisessig erhält man (2R)-2-[3-(4-Amidinophenyl)-2-oxo-5-oxazolidinyl-carbonylamino]-3-phenylpropionsäure-tert.-butylester, Acetat, F. 191-192°.

Analog erhält man
aus 1-[3-(4-lmino-(methylthio)-methylphenyl)-2-oxo-5-oxazolidinylcarbonyl]-piperidin-4-carbonsäure-benzylester:
   1-[3-(4-Amidinophenyl)-2-oxo-5-oxazolidinyl-carbonyl]-piperidin-4-carbonsäurebenzylester, Acetat, F. 197-199°;
aus 2-{1-[3-(4-Imino-(methylthio)-methylphenyl)-2-oxo-5-oxazolidinylcarbonyl]-4-piperidinyloxy}-essigsäure-tert.-butylester:
   2-{1-[3-(4-Amidinophenyl)-2-oxo-5-oxazolidinyl-carbonyl]-4-piperidinyloxy}-essigsäure-tert.-butylester, Acetat, F. 127-126°;
aus 2-{1-[3-(4-Imino-(methylthio)-methylphenyl)-2-oxo-5-oxazolidinylcarbonyl]-4-piperazinyl}-essigsäure-benzylester:
   2-{1-[3-(4-Amidinophenyl)-2-oxo-5-oxazolidinyl-carbonyl]-4-piperazinyl}-essigsäurebenzylester, Acetat.

### Beispiel 13

0,25 g (2R)-2-[3-(4-Amidinophenyl)-2-oxo-5-oxazolidinyl-carbonylamino]-3-phenylpropionsäure-tert.-butylester [erhältlich nach Bsp. 12] werden in 10 ml Trifluoressigsäure bei Raumtemperatur bis zur vollständigen Hydrolyse gerührt. Anschließend engt man die Reaktionsmischung ein und wäscht den Rückstand mehrmals mit Toluol. Nach Behandlung mit Essigsäureethylester erhält man (2R)-2-[3-(4-Amidinophenyl)-2-oxo-5-oxazolidinyl-carbonylamino]-3-phenylpropionsäure, Trifluoracetat, FAB (M+1) 397.

Analog erhält man durch Verseifung der entsprechenden Ester aus Beispiel 12:
1-[3-(4-Amidinophenyl)-2-oxo-5-oxazolidinyl-carbonyl]-piperidin-4-carbonsäure, Trifluoracetat;
2-{1-[3-(4-Amidinophenyl)-2-oxo-5-oxazolidinyl-carbonyl]-4-piperidinyloxy}-essigsäure, Trifluoracetat;
2-{1-[3-(4-Amidinophenyl)-2-oxo-5-oxazolidinyl-carbonyl]-4-piperazinyl}-essigsäure, Trifluoracetat.

### Beispiel 14

23,2 g 3-(4-Cyan-phenyl)-2-oxo-5-oxazolidincarbonsäure [erhältlich nach Bsp. 1] werden zusammen mit 33,0 g Di-tert.-butyl-dicarbonat in 1000 ml Methanol und 110 ml 1 n Natronlauge an Pd-Kohle bis zum Stillstand der Wasserstoffaufnahme hydriert. Anschließend wird die Lösung im Vakuum eingeengt. Nach üblicher Aufarbeitung erhält man 3-(4-tert.-Butoxycarbonylaminomethylphenyl)-2-oxo-5-oxazolidincarbonsäure, F. 166°.

### Beispiel 15

Analog zu Beispiel 4 erhält man durch Umsetzung von 3-(1-Piperazinyl)-propionsäure-benzylester und 3-(4-tert.-Butoxycarbonylaminomethylphenyl)-2-oxo-5-oxazolidincarbonsäure den 3-{1-[3-(4-tert.-Butoxycarbonylaminomethylphenyl)-2-oxo-5-oxazolidinylcarbonyl]-4-piperazinyl}-propionsäurebenzylester, F. 128-130°.

Analog erhält man durch Umsetzung von 3-(4-tert.-Butoxycarbonylaminomethylpheny)2-oxo-5-oxazolidincarbonsäure
mit Piperidin-4-carbonsäurebenzylester:
   1-[3-(4-tert.-Butoxycarbonylaminomethylphenyl)-2-oxo-5-oxazolidinylcarbonyl]-piperidin-4-carbonsäurebenzylester;
mit 2-(4-Piperidinyloxy)-essigsäure-tert.-butylester:
   2-{1-[3-(4-tert.-Butoxycarbonylaminomethylphenyl)-2-oxo-5-oxazolidinylcarbonyl]-4-piperidinyloxy}-essigsäure-tert.-butylester, F. 115-119°;
mit 2-(4-Piperazinyl)-essigsäure-benzylester:
   2-{1-[3-(4-tert.-Butoxycarbonylaminomethylphenyl)-2-oxo-5-oxazolidinylcarbonyl]-4-piperazinyl}-essigsäurebenzylester, F. 121°;
mit 4-Piperidin-carbonsäure-tert.-butylester:
   1-[3-(4-tert.-Butoxycarbonylaminomethylphenyl)-2-oxo-5-oxazolidinylcarbonyl]-4-piperidincarbonsäure-tert.-butylester, F. 63,5°;
mit (2R)-2-Amino-3-phenylpropionsäure-tert.-butylester:
   (2R)-2-[3-(4-tert.-Butoxycarbonylaminomethylphenyl)-2-oxo-5-oxazolidinylcarbonylamino]-3-phenylpropionsäure-tert.-butylester, F. 68-69°.

### Beispiel 16

1,5 g 3-{1-[3-(4-tert.-Butoxycarbonylaminomethylphenyl)-2-oxo-5-oxazolidinylcarbonyl]-4-piperazinyl}-propionsäure-benzylester werden in 50 ml DMF an 5 %iger Pd-Kohle hydriert. Nach üblicher Aufarbeitung wird das Rohprodukt in einem Lösungsmittelgemisch bestehend aus Dichlormethan/Methanol/Eisessig (70:30:2) gelöst und über Kieselgel chromatographiert. Man erhält nach Verreiben des Produktes mit Ether 3-{1-[3-(4-tert.-Butoxycarbonylaminomethylphenyl)-2-oxo-5-oxazolidinylcarbonyl]-4-piperazinyl}-propionsäure, F. 76-78°.

### Beispiel 17

0,53 g 2-{1-[3-(4-tert.-Butoxycarbonylaminomethylphenyl)-2-oxo-5-oxazolidinylcarbonyl]-4-piperidinyl-oxy}-essigsäure-tert.-butylester werden in 10 ml Trifluoressigsäure bei Raumtemperatur gerührt. Nach üblicher Aufarbeitung wird das Rohprodukt mit Ether verrieben und getrocknet. Man erhält 2-{1-[3-(4-Aminomethylpheny)2-oxo-5-oxazolidinylcarbonyl]-4-piperidinyl-oxy}-essigsäure, Trifluoracetat, FAB (M+1): 378.

Analog erhält man
aus (2R)-2-[3-(4-tert.-Butoxycarbonylaminomethylphenyl)-2-oxo-5-oxazolidinylcarbonyl]-3-phenylpropionsäure-tert.-butylester:
(2R)-2-[3-(4-Aminomethylphenyl)-2-oxo-5-oxazolidinylcarbonyl]-3-phenylpropionsäure, Trifluoracetat, F. 178-185°.

### Beispiel 18

0,5 g 1-[3-(4-tert.-Butoxycarbonylaminomethylphenyl)-2-oxo-5-oxazolidinylcarbonyl]-piperidin-4-carbonsäure-tert.-butylester werden in 10 ml Trifluoressigsäure bei Raumtemperatur gerührt. Die Lösung wird im Vakuum eingeengt. Das Rohprodukt wird anschließend mit etherischer Salzsäure behandelt, abgesaugt und getrocknet. Man erhält 1-[3-(4-Aminomethylphenyl)-2-oxo-5-oxazolidinylcarbonyl]-piperidin-4-carbonsäure, Hydrochlorid, F. 256-258° (Zers.).

### Beispiel 19

0,43 g 3-{1-[3-(4-tert.-Butoxycarbonylaminomethylphenyl)-2-oxo-5-oxazolidinylcarbonyl]-4-piperazinyl}-propionsäure, Acetat, werden in 30 ml etherischer HCl-Lösung bei Raumtemperatur gerührt. Der entstandene Niederschlag wird abgesaugt, mit etwas Ether gewaschen und getrocknet. Man erhält 3-{1-[3-(4-Aminomethylphenyl)-2-oxo-5-oxazolidinylcarbonyl]-4-piperazinyl}-propionsäure, Dihydrochlorid, F. 184-186°.

### Beispiel 20

0,74 g 2-{1-[3-(4-tert.-Butoxycarbonylaminomethylphenyl)-2-oxo-5-oxazolidinylcarbonyl]-4-piperazinyl}-essigsäurebenzylester werden in 30 ml HCl-Lösung in Essigsäureethylester bei Raumtemperatur gerührt. Der entstandene Niederschlag wird abgesaugt, mit etwas Essigsäureethylester gewaschen und getrocknet. Man erhält 2-{1-[3-(4-Aminomethylphenyl)-2-oxo-5-oxazolidinylcarbonyl]-4-piperazinyl}-essigsäurebenzylester, Dihydrochlorid, F. 224-226°.

### Beispiel 21

0,6 g 2-{1-[3-(4-Aminomethylphenyl)-2-oxo-5-oxazolidinylcarbonyl]-4-piperazinyl}-essigsäurebenzylester,Dihydrochlorid, werden in einer Mischung aus 30 ml Methanol, 5 ml Wasser und 5 ml Eisessig an Pd-Kohle hydriert. Die Reaktionsmischung wird filtriert und im Vakuum eingeengt. Nach Verreiben des Rohproduktes mit Essigsäureethylester erhält man 2-{1-[3-(4-Aminomethylphenyl)-2-oxo-5-oxazolidinylcarbonyl]-4-piperazinyl}-essigsäure, Dihydrochlorid, F. 91° (Zers.).

### Beispiel 22

5,4 g 3-(4-Aminomethylphenyl)-2-oxo-5-oxazolidincarbonsäure werden in 70 ml THF suspendiert. Unter Rühren werden 35 ml 1 NaOH zugetropft. Danach wird eine Lösung von 6,6 g 4-Cyanbenzoylchlorid in 60 ml THF zugetropft. Der pH der Lösung wird durch Zugabe von 1 n NaOH zwischen 9 und 10,5 gehalten. Nach erfolgter Umsetzung wird mit 2 n HCI bei pH 1 angesäuert. Das Lösungsmittel wird entfernt, der Rückstand abgesaugt und mit Wasser gewaschen.Man erhält 3-[4-(4-Cyanbenzamidomethyl)-phenyl]-2-oxo-5-oxazolidincarbonsäure, F. 234°.

Analog erhält man durch Umsetzung von 3-(4-Aminomethylphenyl)-2-oxo-5-oxazolidincarbonsäure
mit p-Chlorcarbonylaminobenzonitril die
3-{4-[3-(4-Cyanphenyl)-ureidomethyl]-phenyl}-2-oxo-5-oxazolidincarbonsäure.

### Beispiel 23

Analog Beispiel 10 erhält man durch Umsetzung von 3-[4-(4-Cyanbenzamidomethyl)-phenyl]-2-oxo-5-oxazolidinyl-carbonsäure mit Schwefelwasserstoff die 3-[4-(4-Thiocarbamoylbenzamidomethyl)-phenyl]-2-oxo-5-oxazolidincarbonsäure, F. 105-110°.

Analog erhält man durch Umsetzung von 3-{4-[3-(4-Cyanphenyl)-ureidomethyl]-phenyl}-2-oxo-5-oxazolidinylcarbonsäure mit Schwefelwasserstoff die 3-{4-[3-(4-Thiocarbamoylphenyl)-ureidomethyl]-phenyl}-2-oxo-5-oxazolidinylcarbonsäure.

### Beispiel 24

Analog Beispiel 11 erhält man durch Umsetzung von 3-[4-(4-Thiocarbamoyl-benzamidomethyl)-phenyl]-2-oxo-5-oxazolidinyl-carbonsäure mit Methyliodid die 3-[4-(4-Imino-(methylthio)-methyl-benzamidomethyl)-phenyl]-2-oxo-5-oxazolidincarbonsäure, Hydroiodid, F. 209°.

Analog erhält man durch Umsetzung von 3-{4-[3-(4-Thiocarbamoylphenyl)-ureidomethyl]-phenyl}-2-oxo-5-oxazolidincarbonsäure mit Methyliodid die 3-{4-[3-(4-Imino-(methythio)-methylphenyl)-ureidomethyl]-phenyl}-2-oxo-5-oxazolidincarbonsäurebenzylester.

### Beispiel 25

Analog Beispiel 12 erhält man durch Umsetzung von 3-[4-(4-Imino-(methylthio)-methylbenzamido-methyl)-phenyl]-2-oxo-5-oxazolidinylcarbonsäure mit Ammoniumacetat die 3-[4-(4-Amidinobenzamidomethyl)-phenyl]-2-oxo-5-oxazolidincarbonsäure, Acetat, F. 294°.

Analog erhält man durch Umsetzung von 3-{4-[3-(4-Imino-(methylthio)-methylphenyl)-ureidomethyl]-phenyl}-2-oxo-5-oxazolidincarbonsäurebenzylester mit Ammoniumacetat den 3-{4-[3-(4-Amidinophenyl)-ureidomethyl]-phenyl}-2-oxo-5-oxazolidincarbonsäure, Acetat.

### Beispiel 26

Analog Beispiel 21 erhält man durch Abspaltung der Benzylestergruppe ausgehend von 3-[4-(4-Amidinobenzamidomethyl)-phenyl]-2-oxo-5-oxazolidincarbonsäurebenzylester die 3-[4-(4-Amidinobenzamidomethyl)-phenyl]-2-oxo-5-oxazolidincarbonsäure.

Analog erhält man durch Abspaltung der Benzylestergruppe ausgehend von 3-{4-[3-(4-Amidino-phenyl)-ureidomethyl]-phenyl}-2-oxo-5-oxazolidinyl-carbonsäurebenzylester die 3-{4-[3-(4-Amidinophenyl)-ureidomethyl]-phenyl}-2-oxo-5-oxazolidinyl-carbonsäure.

### Beispiel 27

3,36 g 3-(4-tert.-Butoxycarbonylaminomethylphenyl)-2-oxo-5-oxazolidincarbonsäure [erhältlich nach Bsp. 14; F. 166°] werden in einer Mischung aus 30 ml Ether und 30 ml Dioxan, welche zuvor mit HCI-Gas gesättigt wurde, 1 Std. bei Raumtemperatur gerührt. Anschließend wird der Niederschlag abgesaugt und mit Ether gewaschen. Man erhält 3-(4-Aminomethylphenyl)-2-oxo-5-oxazolidincarbonsäure, F. 211-212°.

### Beispiel 28

0,22 g (2R)-2-[3-(4-Cyanphenyl)-2-oxo-5-oxazolidinylcarbonylamino]-3-phenylpropionsäure-tert.-butylester [erhältlich nach Bsp. 8] werden in 5 ml Trifluoressigsäure 1 Std. bei Raumtemperatur gerührt. Anschließend wird die Lösung im Vakuum eingeengt und der Rückstand mit Ether verrieben. Man erhält (2R) -2-[3-(4-Cyanphenyl)-2-oxo-5-oxazolidinylcarbonylamino]-3-phenylpropionsäure, Trifluoracetat.

### Beispiel 29

0,26 g 3-[4-Amino-(hydroxyimino)-methylphenyl]-2-oxo-5-oxazolidincarbonsäure [erhältlich nach Bsp. 2] werden in einer Mischung aus 10 ml Eisessig und 10 ml Essigsäureanhydrid 2 Std. gekocht. Die Reaktionslösung wird im Vakuum eingeengt und der Rückstand aus Ether kristallisiert. Man erhält 3-[4-(5-Methyl-1,2,4-oxadiazol-3-yl)-phenyl]-2-oxo-5-oxazolidincarbonsäure, F. 215-218°.

### Beispiel 30

1,06 g 3-[4-Amino-(hydroxyimino)-methylphenyl]-2-oxo-5-oxazolidincarbonsäure [erhältlich nach Bsp. 2] und 1,47 g Chloracetylchlorid werden in 20 ml Eisessig 2 Std. gekocht. Die Reaktionslösung wird im Vakuum eingeengt und der Rückstand aus Ether kristallisiert. Man erhält 3-[4-(5-Chlormethyl-1,2,4-oxadiazol-3-yl)-phenyl]-2-oxo-5-oxazolidincarbonsäure, F. 178-181°.

### Beispiel 31

1,32 g 3-[4-Amino-(hydroxyimino)-methylphenyl]-2-oxo-5-oxazolidincarbonsäure [erhältlich nach Bsp. 2] und 4,44 g N-(Chlorcarbonylmethyl)-phthalimid werden in 20 ml Eisessig 2 Std. gekocht. Die Reaktionslösung wird auf Raumtemperatur abgekühlt und der Niederschlag abgesaugt, mit Ether und Eisessig gewaschen und aus Ethanol umkristallisiert. Man erhält 3-[4-(5-Phthalimidomethyl-1,2,4-oxadiazol-3-yl)-phenyl]-2-oxo-5-oxazolidincarbonsäure, F. 130-135°.

### Beispiel 32

15 g 3-[4-Amino-(hydroxyimino)-methylphenyl]-2-oxo-5-oxazolidincarbonsäure [erhältlich nach Bsp. 2] werden in 370 ml Eisessig unter Zusatz von 13 ml Essigsäureanhydrid bis zum Stillstand der Wasserstoffaufnahme an Pd-Kohle hydriert Es wird vom Katalysator abfiltriert und das Filtrat verworfen. Anschließend wird der feste Rückstand mit 100 ml 1 n HCl-Lösung und 200 ml konz. HCl-Lösung behandelt und filtriert. Das salzsaure Filtrat wird im Vakuum eingeengt und der sich dabei ausscheidende kristalline Rückstand abfiltriert. Nach Waschen mit wenig Wasser und Trocknung erhält man 3-(4-Amidinophenyl)-2-oxo-5-oxazolidincarbonsäure, Hydrochlorid, F. 253-254°.

### Beispiel 33

0,87 g 1-[3-(4-Cyanphenyl)-2-oxo-5-oxazolidinylcarbonyl]-piperidin-4-carbonsäurebenzylester [erhältlich nach Bsp. 8], 0,43 g Hydroxylammoniumchlorid und 0,89 g Natriumcarbonat werden in 12,5 ml Methanol unter Zusatz von 0,5 ml Wasser 3 Std. gekocht. Die Reaktionslösung wird anschließend wie üblich aufgearbeitet. Man erhält eine Mischung von 1-{3-[4-Amino-(hydroxyimino)-methylphenyl]-2-oxo-5-oxazolidinylcarbonyl}-piperidin-4-carbonsäurebenzylester, FAB (M+1): 467, und 1-{3-[4-Amino-(hydroxyimino)-methylphenyl]-2-oxo-5-oxazolidinylcarbonyl}-piperidin-4-carbonsäure-methylester, FAB (M+1): 391. Die Trennung beider Substanzen erfolgt mittels Säulenchromatographie (Kieselgel; Dichlormethan/Methanol 93:7).

### Beispiel 34

1,12 g 3-{3-[4-(5-Oxo-1,2,4-oxadiazolin-3-yl)-phenyl]-2-oxo-5-oxazolidinylcarbonylamino}-propionsäuremethylester [erhältlich nach Bsp. 4] werden in 13,5 ml Wasser und 30 ml Methanol unter Zusatz von 1,35 g Kaliumcarbonat 3 Std. bei Raumtemperatur gerührt. Die Reaktionslösung wird anschließend wie üblich aufgearbeitet. Man erhält 3-{3-[4-(5-Oxo-1,2,4-oxadiazolin-3-yl)-phenyl]-2-oxo-5-oxazolidinylcarbonylamino}-propionsäure, F. 221-223°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄·2 H₂O, 28,48 g Na₂HPO₄·12 H₂O und 0,1 g Benzalkoniumchlorid in 940 ml zweifach destilliertem Wasser. Man stellt auf pH 6,8 ein, füllt auf 1 auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Oxazolidinoncarbonsäure-Derivate der Formel I 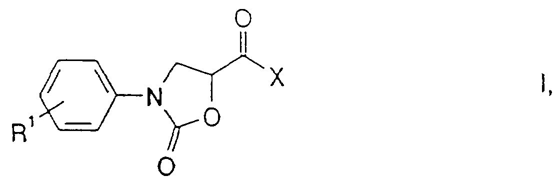 worin
R¹ NO₂, NR⁶R⁷, CN, CONR⁶R⁷, CSNR⁶R⁷, C(=NH)SA, C(=NH)OA, C(=NH)SAr, C(=NH)NHOH, C(=NH)NR⁶R⁷, CH₂NR⁶R⁷, CH₂NHC(=NH)NR⁶R⁷, NHC(=NH)NR⁶R⁷, CH₂NHCO-alk-NR⁶R⁷, CH₂NHCO-Ph-E, CH₂NHCO-Ph-CH₂-NR⁶R⁷, CH₂NHCONH-Ph-E oder D,
x OH, OA, Z, Z-Z', 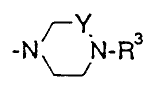 oder 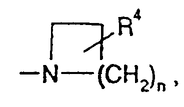
D 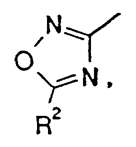
E -CN, -C(=NH)OA, -CSNH₂, -C(=NH)SA oder - C(=NH)NH₂,
Y CH₂, CHOR⁵ oder C=O,
R² H, A, Ar, OH, OA, CF₃, CCl₃, NR⁶R⁷, -alk-NR⁵R⁷, -alk(CH₂Ar)NR⁶R⁷, 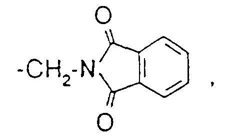
R³ -(CH₂)ₘ-COOR⁵,
R⁴ -(CH₂)ₚ-COOR⁵ oder -(CH₂)_{q}-O-(CH₂)ᵣ-COOR⁵,
Z oder Z' jeweils unabhängig voneinander einen Aminosäurerest, ausgewählt aus einer Gruppe bestehend aus Ala, β-Ala, Arg, Asn, Asp, Gln, Glu, Gly, Leu, Lys, Orn, Phe, Pro, Sar, Ser, Thr, Tyr, Tyr (OMe), Val, C-Allyl-Gly, C-Propargyl-Gly, N-Benzyl-Gly, N-Phenethyl-Gly, N-Benzyl-β-Ala, N-Methyl-β-Ala und N-Phenethyl-β-Ala, wobei freie Amino- oder Carboxyl-Gruppen auch mit an sich bekannten konventionellen Schutzgruppen versehen sein können,
R⁵, R⁶ und R⁷ jeweils unabhängig voneinander H oder A,
m 1, 2 oder 3,
n 1,2, 3 oder 4,
p 0, 1 oder 2,
q 0 oder 1,
r 1 oder 2,
A Alkyl mit 1 bis 6 C-Atomen,
-alk- Alkylen mit 1 bis 6 C-Atomen,
Ar Phenyl oder Benzyl, und
Ph Phenylen
bedeuten,
sowie ihre physiologisch unbedenklichen Salze und/oder Solvate.

2. Enantiomere oder Diastereomere der Verbindungen der Formel I gemäß Anspruch 1.

3. Verbindungen der Formel I gemäß Anspruch 1, worin die freien Amino-, Amidino- oder Guanidinogruppen teilweise oder vollständig durch konventionelle Amino-Schutzgruppen geschützt sind.

4. (a) 2-[1-(3-(4-Amidinophenyl)-2-oxo-5-oxazolidinylcarbonyl)-piperidin-4-yl-oxy]-essigsäure;
(b) (2R)-2-[3-(4-Amidinophenyl)-2-oxo-5-oxazolidinylcarbonylamino]-bemsteinsäure;
(c) 3-[3-(4-Amidinophenyl)-2-oxo-5-oxazolidinylcarbonylamino]-propionsäure;
(d) 1-[3-(4-Amidinophenyl)-2-oxo-5-oxazolidinylcarbonyl]-piperidin-4-carbonsäure-tert.-butylester,
(e) (2R)-2-[3-(4-Amidinophenyl)-2-oxo-5-oxazolidinylcarbonylamino]-bemsteinsäure-di-tert.-butylester,
(f) 1-[3-(4-(5-Oxo-1,2,4-oxadiazolin-3-yl)-phenyl)-2-oxo-5-oxazolidinyl]-piperidin-4-carbonsäure-tert.-butylester;
(g) 3-[4-(Amino(hydroxylimino)methyl)-phenyl]-2-oxo-5-oxazolidincarbonsäure;
(h) (2R)-2-[3-(4-Cyanphenyl)-2-oxo-5-oxazolidinylcarbonylamino]-3-phenylpropionsäure;
(i) 3-[3-(4-(5-Oxo-1,2,4-oxazolidin-3-yl)-phenyl)-2-oxo-5-oxazolidinylcarbonylamino]-propionsäure.

5. Verfahren zur Herstellung von Verbindungen der Formel I gemäß Anspruch 1, **dadurch gekennzeichnet, daß** man
a) eine Verbindung der Formel I aus einem ihrer funktionellen Derivate durch Behandeln mit einem solvolysierenden oder hydrogenolysierenden Mittel in Freiheit setzt, oder daß man
b) eine Verbindung der Formel II 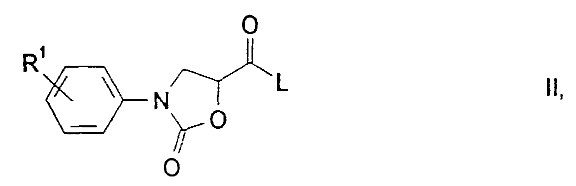 worin
R¹ die angegebene Bedeutung hat und
L Cl, Br, OA, OH oder eine reaktionsfähige veresterte OH-Gruppe bzw. leicht nucleophil substituierbare Abgangsgruppe bedeutet,
mit einer Verbindung der Formel III
H-X' III,
worin X' Z, Z-Z', 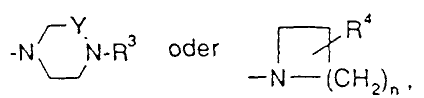 wobei Y, R³, R⁴ und n die angegebenen Bedeutungen besitzen, bedeutet, umsetzt, oder daß man
c) einen Rest X in einen anderen Rest X umwandelt, indem man
- einen Ester der Formel I verseift, oder eine Carbonsäure der Formel 1 veresiert, oder daß man
d) einen Rest R¹ in einen anderen Rest R¹ umwandelt, indem man
- eine NO₂- und/oder CN-Gruppe katatytisch hydriert, oder
- eine Nitrilgruppierung durch Umsetzung mit Ammoniak in eine C(=NH)-NH₂-Gruppe umwandelt, oder
- eine Nitrilgruppe in eine Thiocarbamoylgruppe umwandelt, oder
- eine Thiocarbamoylgruppe in eine Alkylsulfimidogruppe überführt, oder
- eine Carbamoylgruppe in eine Alkylimidogruppe überführt, oder
- eine Methylsulfimidogruppe in eine Amidin-Gruppe überführt, oder
- eine Nitrilgruppe durch Umsetzung mit NH₂OH in eine C(=NH)-NHOH-Gruppe umwandelt, oder
- eine NH₂-Gruppe in eine Guanidinylgruppe umwandelt, oder
- eine C(= NH)-NHOH-Gruppe in eine Amidin-Gruppe umwandelt, oder
- eine CH₂NH₂-Gruppe in eine Alkanoylaminomethyl-, eine CH₂NHC(=NH)NR⁶R⁷-, eine CH₂NHCO-Ph-C(=NH)NR⁶R⁷, eine CH₂NHCO-Ph-CH₂NR⁶R⁷- oder eine CH₂NHCONH-Ph-E-Gruppe umwandelt, oder
- eine 1,2,4-Oxadiazol- bzw. 1,2,4-Oxadiazolinon-Gruppe in eine Amidin-Gruppe umwandelt,
e) oder daß man eine Verbindung der Formel IV 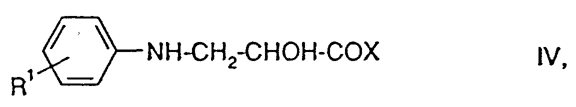 worin
R¹ und X die angegebenen Bedeutungen haben, mit einem reaktionsfähigen Derivat der Kohlensäure umsetzt,
und/oder daß man
f) eine Verbindung der Formel V 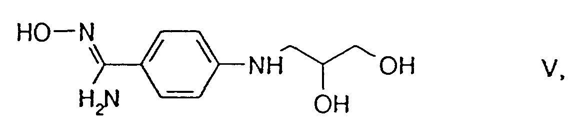 mit 2 Äquivalenten eines reaktionsfähigen Kohlensäurederivates umsetzt und anschließend oxidiert, oder daß man
g) eine Verbindung der Formel I durch Behandeln mit einer Säure oder einer Base in eines ihrer Salze überführt.

6. Verfahren zur Herstellung einer pharmazeutischen Zubereitung, **dadurch gekennzeichnet, daß** man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

7. Pharmazeutische Zubereitung, **gekennzeichnet durch** einen Gehalt an mindestens einer Verbindung der Formel I, gemäß Anspruch 1, und/oder einem ihrer physiologisch unbedenklichen Salze.

8. Verwendung einer Verbindung der Formel I gemäß Anspruch 1 und/oder eines ihrer physiologisch unbedenklichen Salze zur Herstellung von Arzneimitteln.

## Claims

1. Oxazolidinonecarboxylic acid derivatives of the formula I 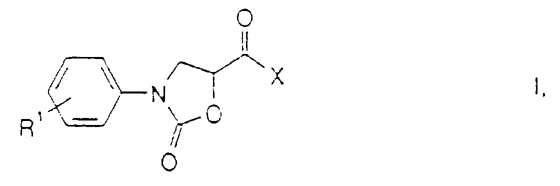 in which
R¹ is NO₂, NR⁶R⁷, CN, CONR⁶R⁷, CSNR⁶R⁷, C(=NH)SA, C(=NH)OA, C(=NH)SAr, C(=NH)NHOH, C(=NH)NR⁶R⁷, CH₂NR⁶R⁷, CH₂NHC(=NH)NR⁶R⁷, NHC(=NH)NR⁶R⁷, CH₂NHCO-alk-NR⁶R⁷, CH₂NHCO-Ph-E, CH₂NHCO-Ph-CH₂NR⁶R⁷, CH₂NHCONH-Ph-E or D,
X is OH, OA, Z, Z-Z', 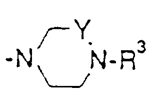 or 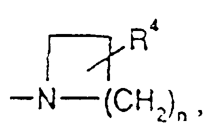
D is 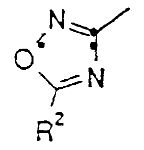
E is -CN, -C(=NH)OA, -CSNH₂, -C(=NH)SA or C(=NH)NH₂,
Y is CH₂, CHOR⁵ or C=O,
R² is H, A, Ar, OH, OA, CF₃, CCl₃, NR⁶R⁷, -alk-NR⁶R⁷, -alk(CH₂Ar)NR⁶R⁷ 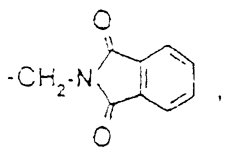
R³ is -(CH₂)ₘ-COOR⁵,
R⁴ is -(CH₂)ₚ-COOR⁵ or -(CH₂)_{q}-O-(CH₂)ᵣ-COOR⁵,
Z or Z' is in each case independent of the other and is an amino-acid residue selected from a group consisting of Ala, β-Ala, Arg, Asn, Asp, Gln, Glu, Gly, Leu, Lys, Orn, Phe, Pro, Sar, Ser, Thr, Tyr, Tyr (OMe), Val, C-allyl-Gly, C-propargyl-Gly, N-benzyl-Gly, N-phenethyl-Gly, N-benzyl-β-Ala, N-methyl-β-Ala and N-phenethyl-β-Ala, it being possible for free amino or carboxyl groups also to be provided with conventional protective groups which are known per se,
R⁵, R⁶ and R⁷ are each independent of one another and are H or A,
m is 1, 2 or 3,
n is 1, 2, 3 or 4,
p is 0, 1 or 2,
q is 0 or 1,
r is 1 or 2,
A is alkyl of 1 to 6 carbon atoms, -alk- is alkylene of 1 to 6 carbon atoms,
Ar is phenyl or benzyl
and
Ph is phenylene,
and their physiologically unobjectionable salts and/or solvates.

2. Enantiomers or diastereomers of the compounds of the formula I according to Claim 1.

3. Compounds of the formula I according to Claim 1, in which the free amino, amidino or guanidino groups are protected in part or in whole by conventional amino-protective groups.

4. (a) 2-[1-(3-(4-amidinophenyl)-2-oxo-5-oxazolidinecarbonyl)piperidin-4-yl-oxy]acetic acid;
(b) (2R)-2-[3-(4-amidinophenyl)-2-oxo-5-oxazolidinylcarbonylamino]succinic acid
(c) 3-[3-(4-amidinophenyl)-2-oxo-5-oxazolidinylcarbonylamino]propionic acid;
(d) tert-butyl 1-[3-(4-amidinophenyl)-2-oxo-5-oxazolidinylcarbonyl]piperidine-4-carboxylate;
(e) di-tert-butyl (2R)-2-[3-(4-amidinophenyl)-2-oxo-5-oxazolidinylcarbonylamino]succinate;
(f) tert-butyl 1-[3-(4-(5-oxo-1,2,4-oxadiazolin-3-yl)phenyl)-2-oxo-5-oxazolidinyl]piperidine-4-carboxylate;
(g) 3-[4-(amino(hydroxylimino)methyl)phenyl]-2-oxo-5-oxazolidinecarboxylic acid;
(h) (2R)-2-[3-(4-cyanophenyl)-2-oxo-5-oxazolidinylcarbonylamino]-3-phenylpropionic acid;
(i) 3-[3-(4-(5-oxo-1,2,4-oxazolidin-3-yl)phenyl)-2-oxo-5-oxazolidinylcarbonylamino]propionic acid.

5. Process for the preparation of compounds of the formula I according to Claim 1, **characterized in that**
a) a compound of the formula I is liberated from one of its functional derivatives by treatment with a solvolysing or hydrogenolysing agent, or **in that**
b) a compound of the formula II 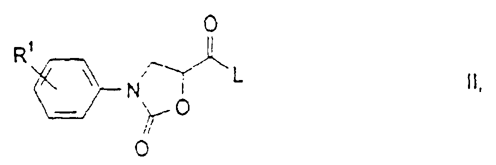 in which
R¹ has the meaning given and
L is Cl, Br, OA, OH or a reactive esterified OH group or a leaving group which is readily capable of undergoing nucleophilic substitution,
is reacted with a compound of the formula III
H-X' III,
in which
X' is Z, Z-Z', 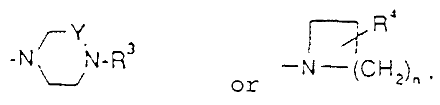 where Y, R³, R⁴ and n have the meanings given, or **in that**
c) a radical X is converted into a different radical X by
- hydrolysing an ester of the formula I or esterifying a carboxylic acid of the formula I, or **in that**
d) a radical R¹ is converted into a different radical R¹ by
- catalytically hydrogenating a NO₂ and/or CN group, or
- converting a nitrile group by reaction with ammonia into a C(=NH)-NH₂ group, or
- converting a nitrile group into a thiocarbamoyl group, or
- converting a thiocarbamoyl group into an alkylsulfimido group, or
- converting a carbamoyl group into an alkylimido group, or
- converting a methylsulfimido group into an amidine group, or
- converting a nitrile group by reaction with NH₂OH into a C(=NH)-NHOH group, or
- converting a NH₂ group into a guanidinyl group, or
- converting a C(=NH)-NHOH group into an amidine group, or
- converting a CH₂NH₂ group into an alkanoylaminomethyl, CH₂NHC(=NH)NR⁶R⁷, CH₂NHCO-Ph-C(=NH)NR⁶R⁷, CH₂NHCO-Ph-CH₂NR⁶R⁷ or a CH₂NHCONH-Ph-E group, or
- converting a 1,2,4-oxadiazole or 1,2,4-oxadiazolinone group into an amidine group,
e) or **in that** a compound of the formula IV 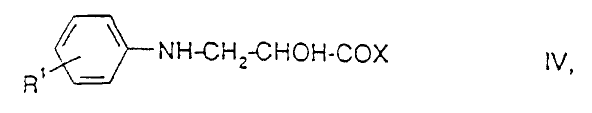 in which
R¹ and X have the given meanings, is reacted with a reactive derivative of carbonic acid,
and/or **in that**
f) a compound of the formula V 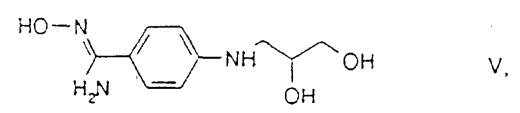 is reacted with 2 equivalents of a reactive carbonic acid derivative and then oxidized, or **in that**
g) a compound of the formula I is converted by treatment with an acid or a base into one of its salts.

6. Process for the preparation of a pharmaceutical formulation, **characterized in that** a compound of the formula I according to Claim 1 and/or one of its physiologically unobjectionable salts is brought, together with at least one solid, liquid or semiliquid excipient or auxiliary, into a suitable administration form.

7. Pharmaceutical formulation, **characterized in that** it contains at least one compound of the formula I according to Claim 1 and/or one of its physiologically unobjectionable salts.

8. Use of a compound of the formula I according to Claim 1 and/or of one of its physiologically unobjectionable salts for the preparation of medicaments.

## Revendications

1. Dérivé de l'acide oxazolidinocarbonique de formule I : 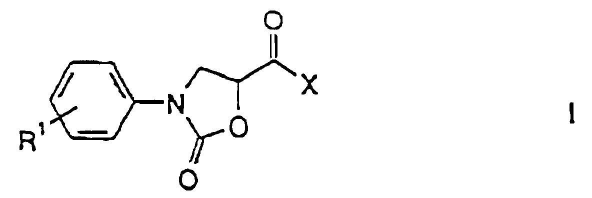 dans laquelle,
R¹ représente NO₂, NR⁶R⁷, CN, CONR⁶R⁷, CSNR⁶R⁷, C(=NH)SA, C-(=NH)OA, C(=NH)SAr, C(=NH)NHOH, C(=NH)NR⁶R⁷, CH₂NR⁶R⁷, CH₂NHC(=NH)NR⁶R⁷, NHC(=NH)NR⁶R⁷, CH₂NHCO-alk-NR⁶R⁷, CH₂-NHCO-Ph-E, CH₂NHCO-Ph-CH₂NR⁶R⁷, CH₂NHCONH-Ph-E ou D.
X représente OH, OA, Z, Z-Z' 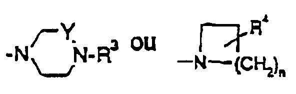
D représente 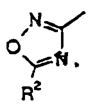
E représente -CN, -C(=NH)OA, -CSNH₂, C(=NH)SA ou -C(=NH)-NH₂
Y représente CH₂, CHOR⁵ ou C=O.
R² représente H, A, Ar, OH, OA, CF₃, CCl₃, NR⁶R⁷, -alk-NR⁵R⁷, -alk-(CH₂Ar)NR⁶R⁷, 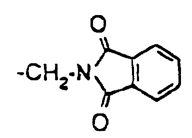
R³ représente -(CH₂)ₘCOOR⁵,
R⁴ représente -(CH₂)ₚCOOR⁵ ou -(CH₂)_{q}-O-(CH₂)ᵣ-COOR⁵,
Z ou Z', chacun indépendamment l'un de l'autre pouvant représenter un résidu d'acide aminé choisi parmi le groupe constitué par Ala, β-Ala, Arg, Asn, Asp, Gln, Glu, Gly, Leu, Lys, Om, Phe, Pro, Sar, Ser, Thr, Tyr, Tyr (OMe), Val, C-Allyl-Gly, C-propargyl-Gly, N-benzyl-Gly, N-phénéthyl-Gly, N-benzyl-β-Ala, N-méthyl-β-Ala et N-phénéthyl-β-Ala, tandis que les groupes amino- ou carboxyle- libres peuvent être munis de groupes protecteurs conventionnels connus en eux-mêmes.
R⁵, R⁶ et R⁷ chacun indépendamment l'un de l'autre représentent H ou A,
m représente 1, 2 ou 3,
n représente 1, 2, 3 ou 4,
p représente 0, 1 ou 2,
q représente 0 ou 1
r représente 1 ou 2,
A représente un alkyle avec 1 à 6 atomes de C -alk- représente un alkylène avec 1 à 6 atomes de C
Ar représente un phényle ou un benzyle, et
Ph représente un phénylène
ainsi que leurs sels et/ou solvants physiologiquement compatibles.

2. Énandomère ou diastéréoisomère des composés de formule 1 selon la revendication 1.

3. Composés de formule 1 selon la revendication 1, dans lesquels les groupes amino-, amidino- ou guanidino libres sont partiellement ou complètement protégés par des groupes protecteurs amino- conventionnels.

4. a) acide 2-[1-(3-(4-amidinophényl)-2-oxo-5-oxazolidinylcarbonyl)-pipéridine-4-yl-oxy]-acétique ;
(b) acide (2R)-2-[3-(4-amldinophényl)-2-oxo-5-oxazolidinylcarbonylamino]-succinique ;
(c) acide 3-[3-(4-amidinophényl)-2-oxo-5-oxazolidinylcarbonylamino]-propionique;
(d) ester tert-butylique de l'acide 1-[3-(4-amidinophényl)-2-oxo-5-oxazolidinylcarbonyl]-pipéridine-4-carbonique;
(e) ester di-tert-butylique de l'acide (2R)-2-[3-(4-amidinophényl)-2-oxo-5-oxazolidinylcarbonylamino]-succinique;
(f) ester tert-butylique de l'acide 1-[3-(4-(5-oxo-1,2,4-oxadiazoline-3-yl)-phényl)-2-oxo-5-oxazolidinyl]-pipéridine-4-carbonique;
(g) acide 3-[4-(amino-(hydroxylimino)-méthyl)-phényl]-2-oxo-5-oxazolidine-carbonique;
(h) acide (2R)-2-[3-(4-cyanophényl)-2-oxo-5-oxazolidinylcarbonylamino]-3-phénylpropionique;
(i) acide 3-[3-(4-(5-oxo-1,2,4-oxazolidine-3-yl)-phényl)-2-oxo-5-oxazolidinylcarbonylamino]-propionique.

5. Procédé de production de composés de formule I selon la revendication 1, **caractérisé en ce que** l'on
a) libère un composé de formule I de l'un de ses dérivés fonctionnels par traitement avec un agent solvolysant ou hydrogénolysant, ou que l'on fait réagir
b) un composé de formule II : 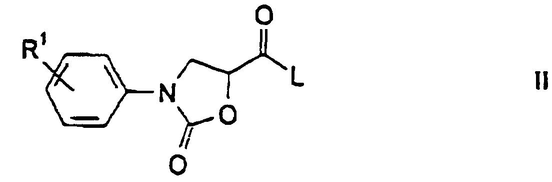 dans laquelle
R¹ a la signification indiquée et
L représente Cl, Br, OA, OH ou un groupe OH- estérifié réactif ou un groupe initial à substitution nucléophile facile, avec un composé de formule III :
H-X' III
dans lequel
X' représente Z, Z-Z', 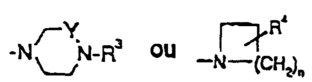 tandis que Y, R³, R⁴ et n ont les significations Indiquées, ou bien on
c) transforme un résidu X en un autre résidu X, dans lequel on
- saponifie un ester de formule 1, ou on estérifie un acide carbonique de formule 1, ou bien on
d) transforme un résidu R¹ en un autre résidu R', dans lequel
- on hydrate catalytiquement un groupe NO₂- et/ou un groupe CN-, ou
- on transforme un groupe nitrile en un groupe C(=NH)-NH₂ par réaction avec de l'ammoniaque, ou
- on transforme un groupe nitrile en un groupe thiocarbamoyle, ou
- on transforme un groupe thiocarbamoyle en un groupe alkylsulfimido, ou
- on transforme un groupe carbamoyle en un groupe alkylimido, ou
- on transforme un groupe méthylsulfimido en un groupe amidine, ou
- on transforme un groupe nitrile en un groupe C(=NH)-NHOH- par réaction avec NH₂OH, ou
- on transforme un groupe NH₂ en un groupe guanidinyl, ou
- on transforme un groupe C(=NH)-NHOH en un groupe amidine, ou
- on transforme un groupe CH₂NH₂ en un groupe alcanoylaminométhyle, en un groupe CH₂NHC(=NH)NR₆R₇, en un groupe CH₂-NHCO-Ph-C(-NH}NR₆R₇, en un groupe CH₂NHCO-Ph-CH₂N-R₆R₇- ou en un groupe CH₂NHCONH-Ph-E-
- on transforme un groupe 1,2,4-oxadiazol ou un groupe 1,2,4-oxadiazolinone en un groupe amidine.
e) ou on transforme un composé de formule IV 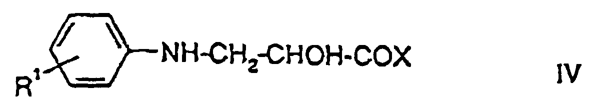 dans laquelle R¹ et X ont les significations indiquées, avec un dérivé réactif du gaz carbonique,
f) et/on on fait réagir un composé de formule V : 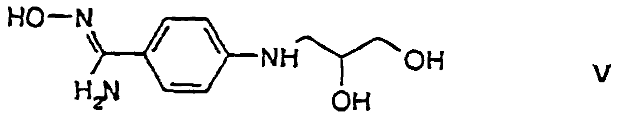 avec 2 équivalents d'un dérivé du gaz carbonique puis on l'oxyde ou bien
g) on transforme un composé de formule I en un de ses sels par traitement avec un acide ou avec une base.

6. Procédé de production d'une préparation pharmaceutique, **caractérisé en ce que** l'on incorpore un composé de formule 1 selon la revendication 1 et/ ou un de ses sels physiologiquement compatibles avec au moins un porteur ou un excipient solide, liquide ou semi-liquide dans une forme d'administration adaptée.

7. Préparation pharmaceutique, **caractérisée par** une teneur en au moins un composé de formule 1, selon la revendication 1, et/ou un de ses sels physiologiquement compatibles.

8. Utilisation d'un composé de formule 1 selon la revendication 1 et/ou d'un de ses sels physlologiquement compatibles pour la production de médicaments.
